# EUROPEAN PATENT APPLICATION

(11) **EP 4 613 172 A1**
(43) Date of publication of application: **10.09.2025**
(21) Application number: 23886275.9
(22) Date of filing: 01.11.2023
(51) Int. Cl.: A47L 23/20, A47L 23/18, H05B 6/54, A61L 2/04, F26B 3/20, A47B 61/04

(54) **ELECTRONIC DEVICE INCLUDING FLOATING ELECTRODE STRUCTURE**

(30) Priority: 01.11.2022 KR 20220143694
(71) Applicant: Samsung Electronics Co., Ltd., Suwon-si, Gyeonggi-do 16677 (KR)
(72) Inventor: PARK, Junhyeong, Suwon-si, Gyeonggi-do 16677 (KR); CHOI, Jinsoo, Suwon-si, Gyeonggi-do 16677 (KR); RYU, Youngho, Suwon-si, Gyeonggi-do 16677 (KR); CHOI, Bohwan, Suwon-si, Gyeonggi-do 16677 (KR)
(74) Representative: Arnold & Siedsma
(86) International application number: PCT/KR2023/017278
(87) International publication number: WO 2024/096579

(57) **Abstract**

An embodiment of the present disclosure may provide an electronic device, the electronic device including: a housing for providing a space capable of receiving an object to be heated; a first electrode and a second electrode for forming an electric field in the space in which the object to be heated is received; and a floating electrode disposed between the first electrode and the second electrode, the floating electrode being positioned inside the object to be heated when the object to be heated is disposed in the space. Various other embodiments may be applied.

## Description

### [Technical Field]

An embodiment of the disclosure relates to an electronic device including a floating electrode structure.

### [Background Art]

Since a dielectric heating method enables energy to quickly and effectively permeate into an object and thus to heat the object so as to sterilize same, the dielectric heating method has been used in a sterilization device or a care device for various product groups including beverages, clothes, and shoes. For example, an electronic device usually called a microwave oven can easily heat an object by radiating energy of a microwave wavelength (e.g., the high frequency of 2.4 GHz to 2.5 GHz), and thus have been widely used.

For example, the dielectric heating method can be applied to electronic devices such as shoe care devices. For example, in a state where two flat plate-type electrodes are positioned on a shelf or in an inner case surrounding a shoe care room of a shoe care device, the shoes accommodated in the shoe care room can be dielectrically heated to dry the shoes and sterilize microorganisms or bacteria inside the shoes. However, in case that shoes to be heated are dielectrically heated in a state of being placed in a shoe care device, due to the low permittivity of shoes and the wide space of a shoe care room designed considering various heights of shoes, the two electrode plates for performing dielectric heating may be formed to be positioned quite far away from each other. Due to this, the electronic devices such as shoe care devices may have low dielectric heating efficiency.

In addition, for example, shoes accommodated in a shoe care device are placed in a case or on a shelf or are accommodated in a form of being mounted by a support member commonly referred to as a shoe tree, and the support member is formed to be attachable thereto or detachable therefrom. Therefore, it is difficult to perform dielectric heating in case of using the support member.

An embodiment of the disclosure, which has been devised to solve the above-described problems, may provide an electronic device including a floating electrode structure, which can easily provide a dielectric heating environment even in case of an electronic device such as a shoe care device and can also improve heating efficiency even in case of an electronic device including an attachable/detachable support member.

### [Detailed Description of the Invention]

### [Technical Solution]

According to an embodiment of the disclosure, provided may be an electronic device including: a housing configured to provide a space capable of accommodating an object to be heated; a first electrode and a second electrode configured to form an electric field in the space in which the object to be heated is accommodated; and a floating electrode disposed between the first electrode and the second electrode, the floating electrode configured to be positioned inside the object to be heated based on the object to be heated being disposed in the space.

According to an embodiment of the disclosure, provided may be an electronic device including: a housing including a case configured to provide a space capable of accommodating an object to be heated, and at least one shelf configured to partition the space capable of accommodating the object to be heated into at least two spaces; a first electrode formed on a portion of the case or a portion of the shelf to form an electric field in the space; a second electrode formed on a portion of the case or a portion of the shelf, which is opposite to the first electrode; a support member configured to support the object to be heated in the space in which the object to be heated is accommodated; and a floating electrode formed on the support member, the floating electrode being disposed between the first electrode and the second electrode and being positioned inside the object to be heated based on the object to be heated being disposed in space.

### [Brief Description of Drawings]

FIG. 1 is a view showing an electronic device including a floating electrode structure according to an embodiment of the disclosure.
FIG. 2A is a perspective view of an electronic device including a floating electrode structure according to an embodiment of the disclosure.
FIG. 2B is a front view of an electronic device including a floating electrode structure according to an embodiment of the disclosure.
FIG. 3 is a view showing a state in which a care function is performed in a state where an object to be heated is mounted to a support member of an electronic device according to an embodiment of the disclosure.
FIG. 4 is a view showing a state in which dielectric heating is performed by applying a voltage to two electrodes in a state where an object to be heated is disposed between the two electrodes.
FIG. 5 is a view showing a state in which a voltage is applied to two electrodes in a state where an object to be heated is disposed between the two electrodes.
FIG. 6 is a view schematically showing distribution of an electric field formed when a voltage is applied thereto and induction heating is performed, in a state where an object to be heated is disposed between two electrodes.
FIG. 7 is a view schematically showing distribution of an electric field formed when a voltage is applied thereto and induction heating is performed, in a state where an object to be heated and a floating structure are arranged between two electrodes.
FIG. 8 is a view schematically showing distribution of an electric field formed when a voltage is applied thereto and induction heating is performed, in a state where an object to be heated is disposed between two electrodes.
FIG. 9 is a view schematically showing distribution of an electric field formed when a voltage is applied thereto and induction heating is performed, in a state where two additional electrodes spaced apart from each other are arranged between two electrodes.
FIG. 10 is a view schematically showing distribution of an electric field formed when a voltage is applied thereto and induction heating is performed, in a state where an object to be heated and a floating structure are arranged between two electrodes according to an embodiment of the disclosure.
FIG. 11 is a view schematically showing distribution of an electric field formed when a voltage is applied thereto and induction heating is performed, in a state where an object to be heated and a floating structure are arranged between two electrodes according to an embodiment of the disclosure.
FIG. 12 is a cross-sectional view showing a state in which a support member is attached to a rail structure according to an embodiment of the disclosure.
FIG. 13 is a view showing a state in which a support member is detached from a rail structure according to an embodiment of the disclosure.

### [Mode for Carrying out the Invention]

Hereinafter, various embodiments of the disclosure will be described with reference to the accompanying drawings.

In the disclosure, the same reference numerals may be used for the same elements. The disclosure may not describe all elements of embodiments, and general contents in the technical field or contents overlapping between embodiments of the disclosure will be omitted.

In the disclosure, a reference numeral for each of steps may be used for convenience of explanation, a reference numeral does not describe the order of steps. Each of steps may be performed in a different order from the described order unless the context clearly describes a specific order.

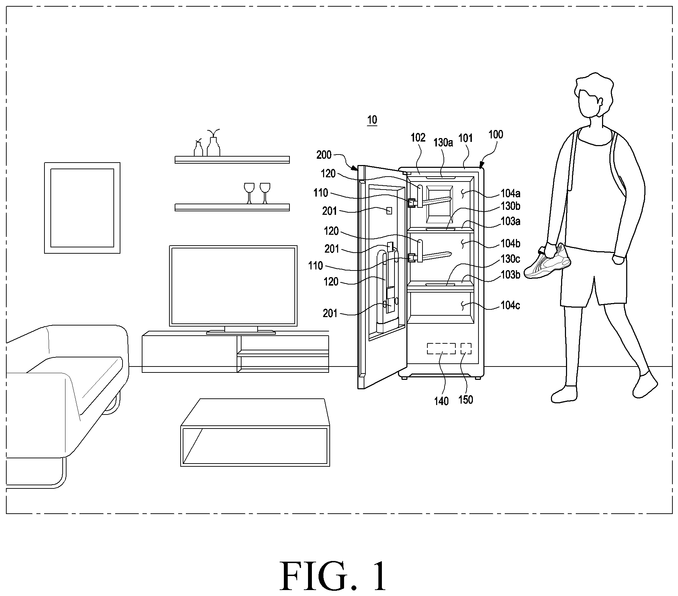

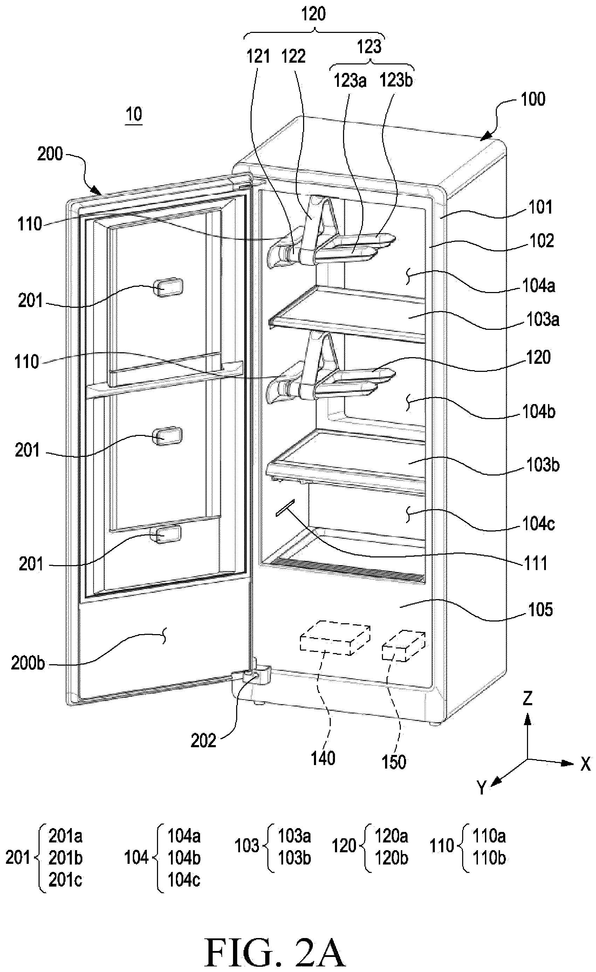

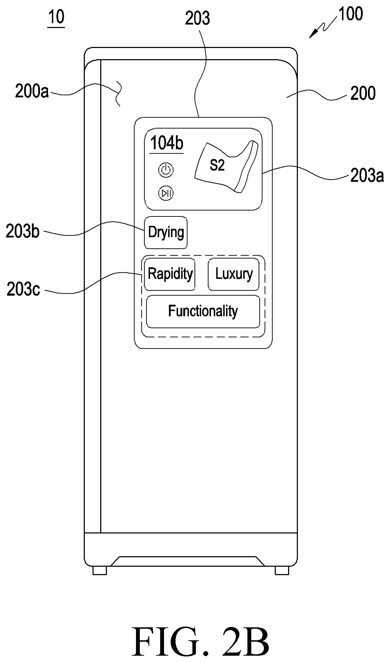

FIG. 1 is a view showing an electronic device 10 including a floating electrode structure according to an embodiment of the disclosure. FIG. 1 is a view showing a state in which shoes are mounted to an electronic device 10 by a user, according to an embodiment of the disclosure. FIG. 2A is a perspective view of an electronic device 10 including a floating electrode structure according to an embodiment of the disclosure. FIG. 2B is a front view of an electronic device including a floating electrode structure according to an embodiment of the disclosure.

In the detailed description below, the vertical width direction of the electronic device 10 may be defined as the "Y-axis direction", the horizontal width direction thereof may be defined as the "X-axis direction", and/or the height direction (the length direction / the vertical direction) thereof may be defined as the "Z-axis direction". In some embodiments, in connection with the direction in which an element is oriented, in addition to the Cartesian coordinate system illustrated in the drawings, "negative/positive (-/+)" may be mentioned together therewith. In connection with the description of directions, when 'negative/positive (-/+)' is not described, it may be interpreted as including both the + direction and the - direction unless otherwise defined. In other words, it may be interpreted that the "X-axis direction" includes both of the +X direction and the -X direction, and it may be interpreted that the "Y-axis direction" includes both of the +Y direction and the -Y direction. In connection with the description of directions, that something is oriented in one axis-direction among the three axis-directions of Cartesian coordinate system may include that something is oriented in the axis-direction and an axis-direction parallel to the axis-direction. The foregoing is based on the Cartesian coordinate system illustrated in the drawings for the sake of brevity of descriptions, and it should be noted that the descriptions of directions or elements do not limit various embodiments of the disclosure.

In the embodiment illustrated in FIG. 1 to FIG. 2C, the electronic device 10 may be a shoe care device (e.g., a shoe dresser). Referring to FIG. 1 to FIG. 2C, the electronic device 10 may include a body 100 forming the exterior thereof and a door 200 rotatably coupled to the body 100. FIG. 1 is a front view showing a state in which a door 200 is opened with respect to a body 100, in a shoe care device, and FIG. 2A is a perspective view showing a state in which a door 200 is opened with respect to a body 100, in a shoe care device, and shows a rear surface 200b of the door 200. FIG. 2B is a front view showing a state in which a door 200 is closed with respect to a body 100, in a shoe care device, and shows a front surface 200a of the door 200.

Referring to FIG. 2A and FIG. 2B, the electronic device 10 may include a body 100 and a door 200 capable of being opened or closed with respect to the body 100. The body 100 may be provided in a rectangular hexahedron shape having an open front surface. The door 200 may be rotatably coupled to the body 100 and be provided to open or close a space 104 (e.g., a care room) which is formed inside the body 100 and can accommodate an object S to be heated. The door 200 may be coupled to the body 100 through a hinge 202. The door 200 may include a hanger 201 provided on a surface thereof, which faces the inside of the space 104 when the space 104 is closed. At least one hanger 201 may be provided. A support member 120 described later may be hung on the hanger 201 and thus be easily stored. The use of the hanger 201 is not limited thereto, and the hanger may also be used in order to store other elements.

The door 200 may further include an input device 203 provided on a front surface of the electronic device 10. According to an embodiment, as illustrated in FIG. 2B, the input device 203 may be disposed on a front surface 200a of the door 200. The electronic device 10 may receive various commands from the user through the input device 203. In addition, a user may set various care courses (e.g., drying, rapidity, luxury, functionality, and the like) through the input device 203, based on the type of shoes to be cared. For example, the input device 203 may include buttons 203b and 203c and a display panel 203a enabling a touch input. The display panel 203a may enable a touch input and may display a progress state of a care course, state information of an object to be heated (or an object to be accommodated) (e.g., shoes), and the like. A user may select the type of shoes accommodated in the space 104 inside the electronic device 10 by using the input device, and set a care course appropriate for the shoes. The display panel 203a may display information and/or icons associated with the electronic device 10. The information and/or icons, which can be displayed on the display panel 203a, may include displaying for the selected inner space (e.g., the second space 104b), On/Off power icon, and Play/Pause icon. However, there is no limitation on displayable information and/or the type, the shape, the size, and the display position of icons. In addition, the display panel 203a may display the state information of scent detected by at least one sensor 106 to be described later. The display panel may display replacement notification of a flavor substance to be described later. Accordingly, a user may easily identify the state information of the electronic device 10 through the display panel without opening the door 200.

Referring to FIG. 2A, the body 100 may include an outer case 101 forming the exterior thereof and an inner case 102 disposed inside the outer case 101. The inner case 102 may form the space 104 (e.g., a care room) capable of accommodating the object S to be heated. The space 104 may be provided to accommodate multiple shoes. A machine room 105 may be provided under the space 104 to supply high-temperature dry air to the space 104. The body 100 may be provided with a pump (e.g., the heat pump 140) and a fluid channel connected to the pump, the pump and the fluid channel allowing air to circulate through the space 104 and the machine room 105. At least one electronic component (e.g., the processor 150) may be disposed in the machine room 105 to control the overall operation of the electronic device 10. For example, a heat pump 140 disposed in the machine room 105 may include a fluid channel, a compressor, a condenser, an expander, an evaporator, other heat exchangers, a blowing fan, and a duct. The operation in which air is circulated by a heat pump system is an obvious matter, and thus the detailed description thereof will be omitted.

The body 100 may include at least one shelf 103 for partitioning the space 104 into at least two spaces. The shelf 103 may be provided inside the body 100 in parallel to the ground. For example, the body 100 may include a first shelf 103a and a second shelf 103b, and the space 104 may be partitioned into a first space 104a, a second space 104b, and a third space 104c by the first shelf 103a and the second shelf 103b. Of course, it should be noted that this is only one embodiment to help understanding and does not limit the number of shelves 103 and spaces 104 of the disclosure. According to the embodiment illustrated in FIG. 2A, although it is illustrated that the space 104 includes three spaces (the first space 104a, the second space 104b, and the third space 104c) capable of individually accommodating the objects S to be heated, it should also be noted that the space may be partitioned into more than 3 spaces or less than 3 spaces. As an example, the shelf 103 provided in the body 100 may be provided to be attachable to or detachable from the body 100. Accordingly, the space 104 capable of accommodating the objects S to be heated in a state where all shelves 103 are attached to the body 100 may also be partitioned into 3 spaces illustrated in FIG. 2A. However, according to another embodiment, at least some shelves 103 may be detached from the body 100, and thus the space 104 capable of accommodating the object S to be heated may also be integrated into two spaces or one space. In case that the space 104 capable of accommodating the object S to be heated is integrated into two spaces or one space in a state where at least some shelves 103 are detached from the body 100, compared to the embodiment having three spaces, the embodiment having two spaces or one space may easily accommodate a long object to be heated (e.g., long boots).

The support member 120 capable of supporting the object to be heated (e.g., shoes) may be provided inside the space 104. The support member 120 may be installed on one side surface of the inner case 102 forming the space 104. Although FIG. 2A illustrates that the support member 120 is positioned at the left side of the space 104, the position of the support member 120 is not limited thereto, and the support member may also be positioned at the right side of the space 104 or at the inner rear side of the space 104. The support member 120 may be provided to be detachable from the body 100. At least one support member 120 may be provided. The support member 120 may be formed in a shape capable of being inserted inside the shoes.

For example, the support member 120 may be the support member 120 having a shoe tree shape for maintaining the shape of an object to be heated (e.g., shoes). The support member 120 may include a base 121 and a support part 122 which extends from one side of the base 121 to restrict a movement of an object to be heated (e.g., shoes). In addition, the support member 120 may include a support frame 123 inserted inside an object to be heated (e.g., shoes) to support the object to be heated (e.g., shoes). The support frame 123 may have a shape which extends in one direction from the base 121 to allow an object to be heated to be supported by the support frame when the object to be heated (e.g., shoes) is inserted inside the support frame. As will be described later, the support frame 123 may perform a function of spraying air for cleaning to care for an object to be heated (e.g., shoes). According to an embodiment, the support frame 123 may include a pair of support frames 123a and 123b, and for example, in case that an object to be heated is shoes, a pair of shoes constituting one set may be inserted into and supported by the pair of support frames 123a and 123b, respectively.

A rail structure 110 for supporting the support member 120 may be provided in the space 104. The support member 120 may be detachably coupled to the rail structure 110. The rail structure 110 may be provided on one side surface of the inner case 102. The rail structure 110 may have a shape which enables the base 121 of the support member 120 to be fitted and coupled therein.

According to an embodiment, the rail structure 110 and the support member 120 may also be provided in all spaces 104 capable of accommodating the object S to be heated, but it is not necessarily limited thereto. That is, the rail structure 110 and the support member 120 may not also be provided in a space (e.g., the third space 104c). For example, as illustrated in FIG. 1 and FIG. 2A, in case that the rail structure 110 and support member 120 are not provided in a space (the third space 104c), it may be possible to care for various types of accommodated objects (e.g., shoes such as baby shoes and sandals which are difficult to be fitted into the support member).

The rail structure 110 may be disposed to correspond to an air supply port (not shown) provided in one side surface of the inner case 102. The rail structure 110 may include an opening (not shown) formed to be connected to the air supply port. Air for cleaning may be supplied through the opening of the rail structure 110 by a pump (e.g., the heat pump 140) disposed in the machine room 105 of the body 100, so as to deodorize and/or dry an object to be heated (e.g., shoes).

According to an embodiment of the disclosure, the electronic device 10 may include a "floating electrode disposing structure" including a first electrode, a second electrode, and a third electrode (hereinafter, referred to as a "floating electrode") disposed between the first electrode and the second electrode, and thus the electronic device 10 can evenly heat (sterilize) an object regardless of the type or size of the object without expensive components provided therein. In the following embodiment, for the convenience of explanation, as a type of object, shoes requiring sterilization may be used as an example, but it should be noted that it is not limited thereto.

The floating electrode disposing structure of the disclosure may be based on the induction heating or the dielectric heating method, and may be configured such that, when an electric field is formed around an object, the polar molecules of the object rotate or vibrate, and the frictional heat generated at this time is used to heat the object. For example, in case that the floating electrode disposing structure is applied to shoes requiring sterilization, the useful life of shoes is greatly increased. The electronic device 10 including a floating electrode structure of the disclosure may be configured to heat an object by using an alternating electric current having a frequency of several MHz to several tens MHz, thereby uniformly heating the outside and the inside of the object.

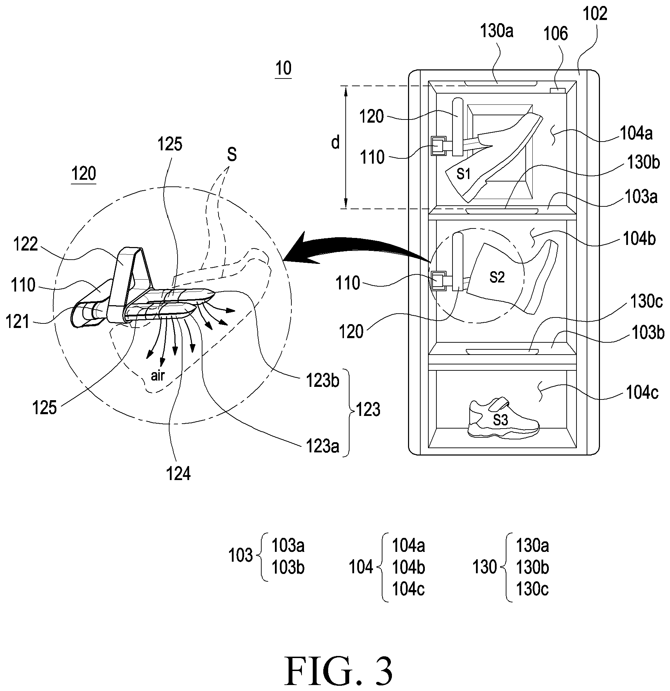

Some (a first electrode and a second electrode) of electrodes included in the floating electrode disposing structure may be provided in the inner case 102 or shelf 103 of the electronic device 10 as illustrated in FIG. 1. According to an embodiment, some (a first electrode and a second electrode) of electrodes included in the floating electrode disposing structure may be provided in each of partitioned spaces 104. That is, the electronic device 10 may include a plurality of floating electrode disposing structures. For example, the electronic device 10 in FIG. 1 may include a first floating electrode disposing structure, a second floating electrode disposing structure, and/or an additional floating electrode disposing structure. For example, the electronic device 10 may include a first electrode 130a disposed on the upper part inside the inner case 102, a second electrode 130b disposed on the first shelf 103a, and a floating electrode (the floating electrode 125 described later in FIG. 3) disposed between the first electrode 130a and the second electrode 130b, to form one floating electrode disposing structure (e.g., a first floating electrode disposing structure). As another example, the electronic device 10 may include a first electrode 130b disposed on a first shelf 103a, a second electrode 130c disposed on a second shelf 103b, and a floating electrode (the floating electrode 125 described later in FIG. 3) disposed between the first electrode 130b and the second electrode 130c, to form one floating electrode disposing structure (e.g., a second floating electrode disposing structure). According to an embodiment, electrodes (e.g., 130b) disposed on one shelf may be used together in floating electrode disposing structures different from each other. Hereinafter, in explaining various embodiments, for the convenience of explanation, it will be described focusing on a first floating electrode disposing structure including a first electrode 130a disposed on the upper surface of the inner case 102, a second electrode 130b disposed on the first shelf 103a, and a floating electrode (the floating electrode 125 described later in FIG. 3) disposed between the first electrode 130a and the second electrode 130b. It should be noted that this can also be applied to other floating electrode disposing structures.

FIG. 3 is a view showing a state in which a care function is performed in a state where an object to be heated is mounted to a support member of an electronic device according to an embodiment of the disclosure.

Referring to FIG. 3, the electronic device 10 of the disclosure may have a structure which allows accommodated objects (e.g., S1, S2, S3) having various types and/or sizes to be easily cared. The electronic device 10 may care for some accommodated objects S1 and S2 in a state where the objects are raised from above the floor (e.g., the shelf) of the space 104 by using a support member 120, and may care for other accommodated objects S3 in a state where the objects are placed on the floor (e.g., a shelf or an inner case) of the space 104 without using a support member 120. The disclosure may provide the electronic device 10 which allows some accommodated objects S1 and S2 to be cared for in a state where the objects are raised from above the floor (e.g., the shelf) of the space 104 by using a support member 120, the accommodated objects being an objects to be heated.

The support member 120 may provide general care for an object to be heated (e.g., shoes S) through the support frame 123 inserted into the object to be heated (e.g., shoes S). Here, for example, "general care" may mean that an object to be heated is cared for in a manner of spraying air for cleaning. According to an embodiment, the high-temperature dry air supplied from the heat pump 140 may dry and/or dehumidify an object to be heated. According to an embodiment, the support member 120 may include two support frames 123a and 123b which can be inserted into a pair of shoes, respectively, and an opening 124 may be formed through each of the support frame 123a, 123b to discharge air downward and thus dry and/or dehumidify an object to be heated. The electronic device 10 may perform a drying and/or a dehumidifying function, and additionally or alternatively, may also perform a deodorizing and/or a sterilizing function. According to another embodiment, the electronic device 10 may further include a filter (e.g., a UV filter (not shown)), and the filter may also perform a function of removing odor by decomposing odor particles separated from an object to be heated by the air for cleaning.

The disclosure may provide a floating electrode structure to provide an improved heating function for an object to be heated, in addition to the above-described general care function by using the support member 120.

As illustrated in FIG. 3, in a state where the first electrode 130a is disposed in the inner case 102 and the second electrode 130b is disposed on a shelf (e.g., the first shelf 103a), the object S to be heated, which is disposed in the space 104, may be induction-heated even only by the first electrode 130a and the second electrode 130b. However, in this case, since the distance d between the first electrode 130a and the second electrode 130b is far, it may be difficult to sufficiently obtain the effects by induction heating. For example, in case that an object to be heated is the shoes S, the shoes may have various heights according to the type of shoes. Therefore, the space 104, in which the shoes are accommodated, should be formed to have a large volume. Accordingly, the first electrode 130a and the second electrode 130b, which are spaced apart from each other in the inner case 102 and/or on the shelf 103, may be formed to be spaced apart from each other by a distance corresponding to the inner case 102 having a large volume and the shelf 103 and/or the distance between shelves. In addition, since the shoes S are usually formed of fibers such as linen and cotton, or materials such as cork, urethane, polyester, leather, and the like having low permittivity, it may be difficult to obtain the effects by induction heating.

The electronic device 10 including a floating electrode structure of the disclosure may perform a heat function by using a floating electrode disposed between an electrode and an electrode, to significantly improve the heating effect of an object to be heated, especially the internal heating effect of an object to be heated.

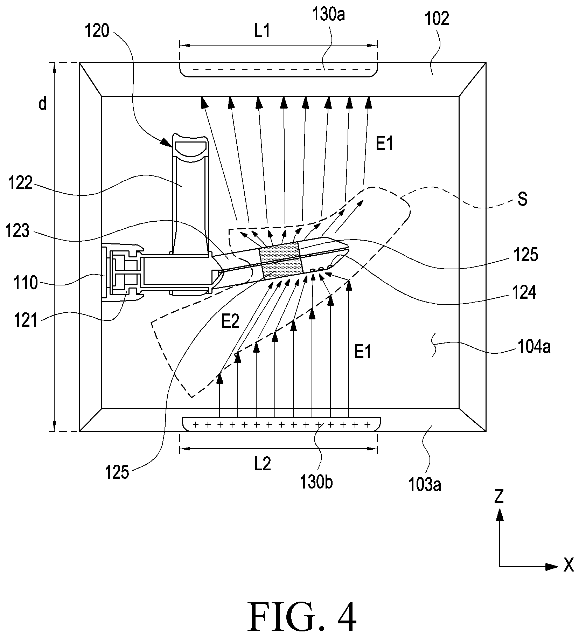

Hereinafter, the internal heating effect by the floating electrode structure provided in the disclosure will be described in detail with reference to the embodiments of FIG. 4 to FIG. 11.

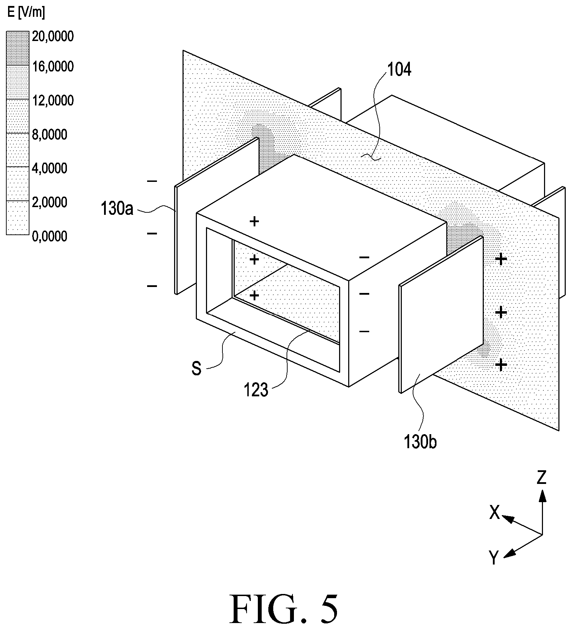

FIG. 4 is a view showing a state in which dielectric heating is performed by applying a voltage to two electrodes in a state where an object to be heated is disposed between the two electrodes. FIG. 5 is a view showing a state in which a voltage is applied to two electrodes in a state where an object to be heated is disposed between the two electrodes.

The electronic device 10 may include a first electrode 130a and a second electrode 130b which are spaced apart from each other in the inner case 102 and/or on the shelf 103. The first electrode 130a and the second electrode 130b may be arranged at positions corresponding to each other in the inner case 102 and/or on the shelf 103. The first electrode 130a and the second electrode 130b may be flat plate-type electrodes and may have a width and a length of several tens mm. According to an embodiment, the first electrode 130a and the second electrode 130b may have substantially the same width and length. For example, the width L1 of the first electrode 130a and the width L2 of the second electrode 130b may be substantially the same. The electronic device 10 may perform dielectric heating by using the first electrode 130a and the second electrode 130b. Here, in case that an object to be heated is positioned in a high frequency electric field formed by the first electrode 130a and the second electrode 130b, dielectric heating may be implemented based on the principle that frictional heat loss occurs in the molecules of the object to be heated and thus the object to be heated is heated.

As mentioned above, in case that an object to be heated is the shoes S, the distance d between the first electrode 130a and the second electrode 130b may be significantly far. The first electrode 130a and the second electrode 130b may be formed to be spaced apart from each other by several hundred mm, for example, approximately 120 mm. In case that the distance between the first electrode 130a and the second electrode 130b is far, it may be difficult to effectively perform dielectric heating, and also since the permittivity of an object to be heated is low, it may be difficult to achieve a high dielectric heating effect. In the disclosure, in order to solve the problems, a floating electrode 125 may be provided on the support member 120 included in the electronic device 10 to improve the dielectric heating effect.

The support member 120 may be spaced apart from the inner case 102 and/or a shelf (e.g., the first shelf 103a), and may be positioned approximately at the center of a space (e.g., the first space 104a) between the inner case 102 and/or the shelf (e.g., the first shelf 103a). One end (e.g., the base 121) of the support member 120 may be fixed to the rail structure 110, and in case that the one end (e.g., the base 121) of the support member 120 is fixed, the other end (e.g., the support frame 123) thereof may be disposed to protrude to a space (e.g., the first space 104a). According to an embodiment, the support member 120 may include various materials, but may be formed as a rigid body as a whole to stably support an object to be heated (e.g., the shoes S). Therefore, even in case that an object to be heated (e.g., the shoes S) is mounted to the support member, the support member 120 may stably support the object to be heated (e.g., the shoes S) while preventing the object to be heated (e.g., the shoes S) from sagging by the weight. In case that a heated object mounted to the support member 120 is the shoes S, the tongue portion or the toe cap portion of the shoes S may be positioned adjacent to the support frame 123 by inserting the shoes S into the support frame 123 of the support member 120. Alternatively, the sole (e.g., including the insole, outsole, and midsole) of the shoes S may also be positioned adjacent to the support frame 123.

According to an embodiment, a floating electrode 125 may be provided on the support member 120 of an electronic device 10. For example, the floating electrode 125 may be disposed on the support frame 123 of the support member 120. For another example, in case that a heated object mounted to the support member 120 is the shoes S, the floating electrode 125 on the support frame 123 of the support member 120 may be positioned at a position which at least partially overlaps a position adjacent to the tongue portion, the toe cap portion, and/or the sole portion of the shoes S. Accordingly, in case that an object to be heated (e.g., the shoes S) is mounted to the support member 120, the floating electrode 125 may be substantially surrounded by the object to be heated (e.g., the shoes S).

The floating electrode 125 may be configured to be electrically insulated from the first electrode 130a and the second electrode 130b. In addition, the floating electrode 125 may be configured to be disposed on the support frame 123 of the support member 120 in a state of being electrically insulated from other components. In addition, the support frame 123 may be formed of a high insulation material such as polycarbonate or an elastic material (e.g., urethane), and be insulated from the floating electrode 125. The floating electrode 125 may be a passive element other than an active element which implements a function by itself upon receiving a command from the processor 150 or upon satisfying a set condition. Therefore, in a state of being electrically insulated from other elements, without an applied electrical input or signal, the floating electrode 125 may cause the effect of the dielectric heating function to be improved in conjunction with operations of other elements (e.g., the first electrode 130a and the second electrode 130b).

When the floating electrode 125 is viewed from above or below, the floating electrode 125 can be formed at a position overlapping the first electrode 130a and the second electrode 130b. The floating electrode 125 may be positioned between the first electrode 130a and the second electrode 130b and be positioned at a position at least partially overlapping the first electrode 130a and the second electrode 130b, and thus the floating electrode may be positioned in an electric field E1 formed by the first electrode 130a and the second electrode 130b. According to an embodiment, by the electric field E1 formed by the first electrode 130a and the second electrode 130b, the floating electrode 125 disposed between the first electrode 130a and the second electrode 130b may be induced. Referring to FIG. 4, in case that an object to be heated (e.g., the shoes S) is positioned between the first electrode 130a and the second electrode 130b in a state where a voltage is applied to the first electrode 130a and the second electrode 130b and thus the electric field E1 is formed, the electric field E1 may be at least partially blocked by the object to be heated (e.g., the shoes S). At this time, the electric field E1 between the first electrode 130a and the second electrode 130b may be distorted by the object to be heated (e.g., the shoes S) having a predetermined permittivity. In viewpoint of an object to be heated (e.g., the shoes S), since an electric field (hereinafter, referred to as "external electric field E1" with reference to an object to be heated) is formed therearound, vibration of molecules by dielectric heating may occur on the surface of an object to be heated (e.g., the shoes S). In a state where an object to be heated (e.g., the shoes S) is mounted to the support member 120 and the floating electrode 125 is substantially surrounded by the object to be heated (e.g., the shoes S), a charge may be induced in the floating electrode 125 by the external electric field E1 formed by the first electrode 130a and the second electrode 130b. For example, the floating electrode 25 may be a conductive body formed of a metal material such as copper; in case that the floating electrode is affected by the external electric field E1, one side of the floating electrode is induced to have a positive (+) charge and the other side thereof is induced to have a negative (-) charge; and in case that the external electric field E1 causing the influence thereof disappears, the floating electrode 25 may be returned to the original state thereof. The charges induced on the floating electrode 125 may form an internal electric field E2 in the space between an object to be heated (e.g., the shoes S) and the floating electrode 125. The dielectric heating effect for an object to be heated (e.g., the shoes S) may be significantly improved by the internal electric field E2. In summary, according to the disclosure, a conductive body (a floating electrode) may be disposed between two flat plate-type electrodes (the first electrode 130a and the second electrode 130b) which are physically and/or electrically spaced apart from each other, a voltage may be applied to the two flat plate-type electrodes (the first electrode 130a and second electrode 130b) to induce charge in the conductive body (a floating electrode), and thus the distance (hereinafter, referred to as "effective distance") between electrodes to dielectrically heat the two flat plate-type electrodes (the first electrode 130a and the second electrode 130b) can be reduced.

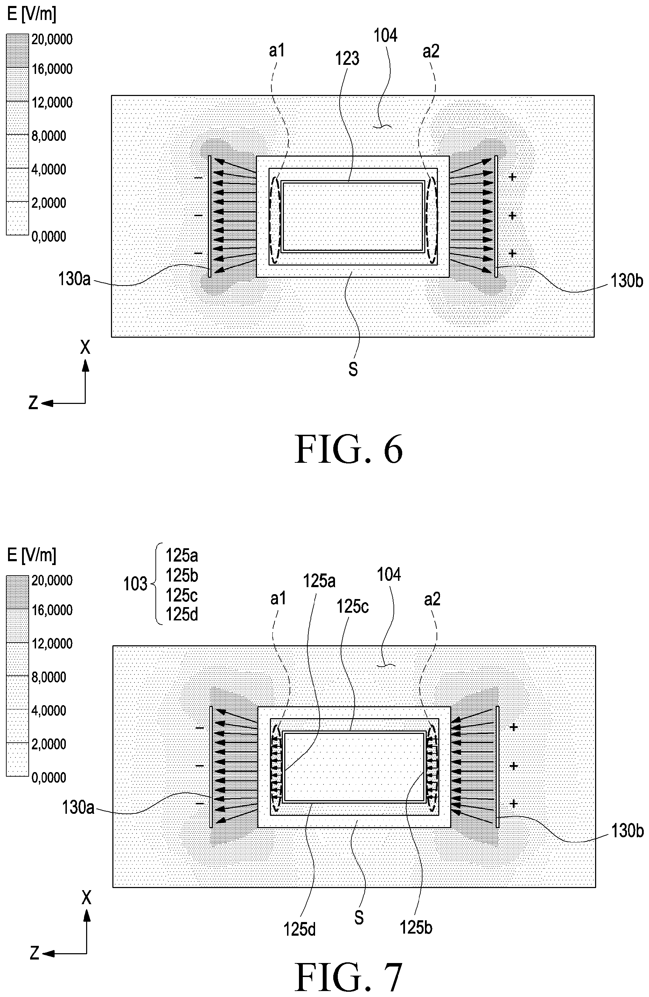

In case that the floating electrode 125 is physically spaced apart from the first electrode 130a and the second electrode 130b, the floating electrode may have various specific shapes and positions according to embodiments. As will be described in detail later through the embodiment of FIG. 7 and below, the floating electrode 125 of the disclosure may include a portion at least partially facing the first electrode 130a (e.g., the first portion 125a in FIG. 7) and a portion facing the second electrode 130b (e.g., the second portion 125b in FIG. 7), and the portion facing the first electrode 130a (e.g., the first portion 125a in FIG. 7) and the portion facing the second electrode 130b (e.g., the second portion 125b in FIG. 7) may be formed to be electrically connected to each other. The floating electrode 125 may have a shape corresponding to the shape of the support frame 123. For example, as illustrated in FIG. 3, in case that the support frame 123 has an elongated rod shape having a flat elliptical cross section, the floating electrode 125 may have a cylindrical shape having a flat elliptical cross section. For another example, the floating electrode 125 may also be formed in a ring cross section shape or a loop cross section shape. For another example, the floating electrode 125 may also have a column shape having a quadrilateral cross section as illustrated in FIG. 5. In addition, the floating electrode 125 may have various cross section shapes such as a circle, a triangle, a polygon other than a square, and the like. For another example, the floating electrode 125 may also have various polyhedral shapes including a hexahedral shape as well as a shape having a through-type opening as illustrated in drawings. For another example, the floating electrode 125 may also have a shape in which a conductive body is rolled.

In the embodiment of FIG. 3 and FIG. 4, although it is illustrated that the floating electrode 125 is formed along the circumference of the support frame 123, it is not limited thereto. For example, the floating electrode 125 may also have an elongated shape extending the direction in which the support frame 123 extends. In addition, in the embodiments of FIG. 3 and FIG. 4, although it is illustrated that the floating electrode 125 is formed on the surface of the support frame 123 to be exposed to the outside, it should also be noted that the floating electrode 125 is formed inside the surface of the support frame 123 so as not to be exposed to the outside.

The floating electrode 125 may also be implemented as a single floating electrode or multiple floating electrodes. For example, the floating electrode 125 disposed between the first electrode 130a and the second electrode 130b may also be provided as a single floating electrode 125 or a pair of floating electrodes 125 respectively arranged on the pair of support frames 123 as illustrated in FIG. 3, and a greater number of floating electrodes, which are not illustrated in the drawings, may be provided. FIG. 6 is a view schematically showing distribution of an electric field formed when a voltage is applied thereto and induction heating is performed, in a state where an object to be heated is disposed between two electrodes. In the embodiments of FIG. 6 and below described below, the cross sections of an object to be heated (e.g., the shoes S), the support frame 123, and/or the floating electrode 125 may be simply represented as a Z-axis direction component, an X-axis direction component, and a direction component parallel thereto, but it should be noted that this is for convenience of explanation.

FIG. 6 shows a simulation result of an electric field formed around an object to be heated (e.g., the shoes S) and the support frame 123 in a state where the support frame 123 of the support member 120 is inserted inside an object to be heated (e.g., the shoes S). In the embodiment of FIG. 6, in case that the distance between the first electrode 130a and the second electrode 130b is set to 120 mm and an object to be heated (e.g., the shoes S) has a cross section of 80 mm in the Z-axis direction and 50 mm in the X-axis direction, the simulation result where a voltage is applied to the first electrode 130a and the second electrode 130b is illustrated.

FIG. 6 shows the support frame 123 not provided with the floating electrode 125. Here, the support frame 123 may be formed of an insulation material. Referring to FIG. 6, in an embodiment where the floating electrode 125 is not provided, in case that a predetermined voltage is applied to the first electrode 130a and the second electrode 130b, it is shown that an electric field having a high density (approximately 20 V/m) is formed outside the surface of an object to be heated (e.g., the shoes S), with reference to the object to be heated (e.g., the shoes S). On the other hand, it is shown that an electric field having a significantly lower density is formed inside the surface of the object to be heated (e.g., the shoes S), for example, in the spaces a1 and a2 between the object to be heated (e.g., the shoes S) and the support frame 123, compared to the outer surface.

FIG. 7 is a view schematically showing distribution of an electric field formed when a voltage is applied thereto and induction heating is performed, in a state where an object to be heated and a floating structure are arranged between two electrodes. FIG. 7 shows a simulation result of an electric field formed when a voltage is applied to the first electrode 130a and the second electrode 130b, when all conditions are the same except that the floating electrode 125 is additionally disposed inside the object to be heated (e.g., the shoes S) compared to FIG. 6. In FIG. 7, a support frame (e.g., the support frame 123 in FIG. 6) is omitted. In the embodiment of FIG. 7, the first electrode 130a and the second electrode 130b may be flat plate-type electrodes as the embodiment illustrated in FIG. 6. Even in the embodiment of FIG. 7, in case that the distance between the first electrode 130a and the second electrode 130b is set to 120 mm and an object to be heated (e.g., the shoes S) has a cross section of 80 mm in the Z-axis direction and 50 mm in the X-axis direction, the simulation result where a voltage is applied to the first electrode 130a and the second electrode 130b is illustrated. In the embodiment of FIG. 7, as a floating electrode 125, a floating electrode 125 having a quadrilateral cross section is additionally illustrated. The floating electrode may have a cross section of 60 mm in the Z-axis direction and 30 mm in the X-axis direction.

Referring to the embodiment illustrated in FIG. 7, in case that a predetermined voltage is applied to the first electrode 130a and the second electrode 130b, it is shown that an electric field having a high density (e.g., approximately 20 V/m) is formed outside the surface of an object to be heated (e.g., the shoes S), with reference to the object to be heated (e.g., the shoes S). In addition, it is shown that an electric field having a relatively high density compared to FIG. 6 is formed inside the surface of the object to be heated (e.g., the shoes S), for example, in the spaces a1 and a2 between the object to be heated (e.g., the shoes S) and the support frame 123.

According to an embodiment, as illustrated in FIG. 7, the floating electrode 125 may include a first portion 125a at least partially facing the first electrode 130a and a second portion 125b facing the second electrode 130b. In addition, the first portion 125a facing the first electrode 130a and the second portion 125b facing the second electrode 130b may be formed to be electrically connected to each other. For example, the floating electrode 125 may include a third portion 125c and/or a fourth portion 125d for connecting the first portion 125a and the second portion 125b. Although FIG. 7 shows a floating electrode 125 having a quadrilateral cross section as an example, the shape of the floating electrode 125 is not limited thereto. Accordingly, the floating electrode 125 may also further include a fifth portion which is different from the first portion 125a to the fourth portion 125d. In addition, differently from that illustrated in FIG. 7, each of the first portion 125a to the fourth portion 125d may also include at least a part formed in a curved surface.

In the embodiment of FIG. 6 and FIG. 7, in case that the distance between the first electrode 130a and the second electrode 130b is set to 120 mm and an object to be heated has a cross section of 80 mm in the Z-axis direction and 50 mm in the X-axis direction, the simulation result where a voltage is applied to the first electrode 130a and the second electrode 130b is illustrated. In the embodiment of FIG. 6, an electric field of about 8 volt/meter (V/m) or less may be formed in the spaces a1 and a2 between an object to be heated (e.g., the shoes S) and the support frame 123. Differently therefrom, in the embodiment of FIG. 7, an electric field exceeding 12 volt/meter (V/m) may be formed in the space a1 between an object to be heated (e.g., the shoes S) and the first portion 125a of the floating electrode 125, and in the space a2 between the object to be heated (e.g., the shoes S) and the second portion 125b of the floating electrode 125. According to an embodiment, compared to the embodiment of FIG. 6, the embodiment of FIG. 7 can obtain the effect in which the electric field is strengthened by more than three times in the inner space of the surface of an object to be heated (e.g., the shoes S). By adding the floating electrode 125, it is identified that an electric field is strengthened inside the object S to be heated by the charge induced in the floating electrode 125.

In the disclosure, in case that an object to be heated (e.g., the shoes S) is disposed in the space 104, the floating electrode 125 positioned inside the object S to be heated is included therein, and thus the internal heating effect of the object S to be heated can be significantly increased using the effect in which the effective distance forming an electric field between electrodes (the first electrode 130a and the second electrode 130b) is reduced.

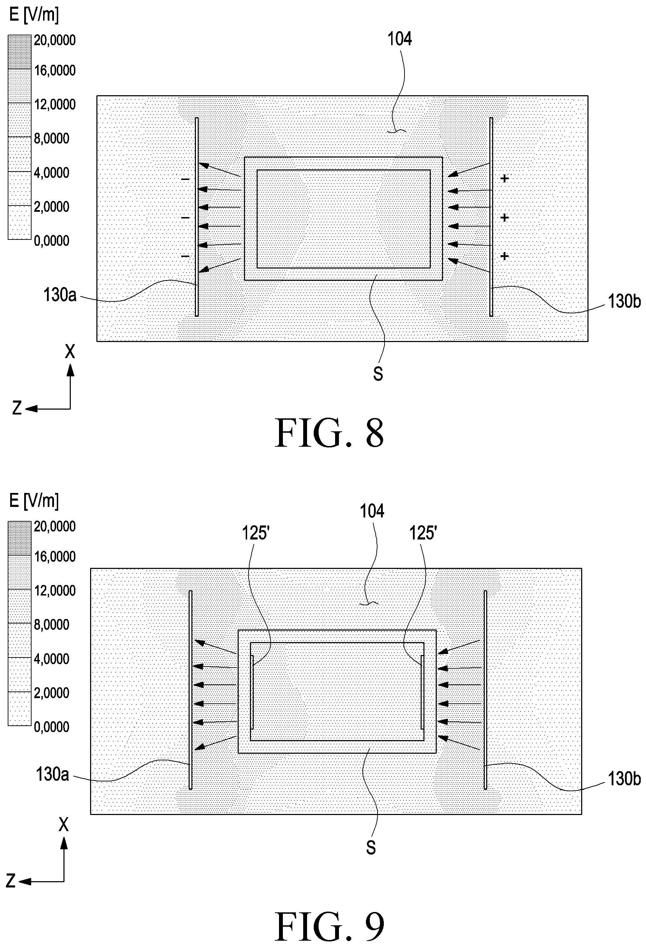

Hereinafter, the floating electrode structure will be described in more detail with reference to the embodiments of FIG. 8 to FIG. 11. As previously described, FIG. 8 to FIG. 11 show simulation results of electric field distribution in the space 104 of a shoe care device, in which an object to be heated is accommodated.

FIG. 8 is a view schematically showing an electric field formed when a voltage is applied thereto and induction heating is performed, in a state where an object to be heated is disposed between two electrodes.

FIG. 8 shows a simulation result of electric field distribution formed in the space 104 between the object S to be heated and the first electrode 130a and between the object to be heated and the second electrode 130b. At this time, the first electrode 130a and the second electrode 130b may be flat plate-type electrodes.

FIG. 8 shows a state in which an electric field is formed around an object to be heated, in an embodiment not provided with a floating electrode. Referring to FIG. 8, the first electrode 130a may be a negative (-) electrode and the second electrode 130b may be a positive (+) electrode. Differently from that illustrated in FIG. 8, it is also possible that the first electrode 130a is formed as a positive electrode and the second electrode 130b is formed as a negative electrode.

Differently from the embodiment of FIG. 6, the embodiment of FIG. 8 may not have a separate support frame 123 inside an object to be heated (e.g., the shoes S). In addition, FIG. 8 shows an electric field generated when an object to be heated (e.g., the shoes S), which has permittivity different from that of the object to be heated (e.g., the shoes S) of the embodiment of FIG. 6, is disposed between the first electrode 130a and the second electrode 130b.

Referring to FIG. 8, in an embodiment not provided with the floating electrode 125, in case that a predetermined voltage is applied to the first electrode 130a and the second electrode 130b, it is identified that the electric field formed between the first electrode 130a and the second electrode 130b has a value of approximately 4 to 12 volt/meter (V/m). In the embodiment of FIG. 8, it is identified that the influence of an object to be heated (e.g., the shoes S) on the electric field is insignificant.

FIG. 9 is a view schematically showing an electric field formed when a voltage is applied thereto and induction heating is performed, in a state where two additional electrodes spaced apart from each other are arranged between two electrodes.

In addition, FIG. 9 shows a simulation result of an electric field formed in the space 104 between the object S to be heated and the first electrode 130a and between the object to be heated and the second electrode 130b. At this time, the first electrode 130a and the second electrode 130b may be flat plate-type electrodes, a flat plate-type floating electrode 125' as a floating electrode may be disposed between the first electrode 130a and the second electrode 130b. Two flat plate-type floating electrodes 125' may be arranged inside the object S to be heated, one thereof may be disposed close to the first electrode 130a and thus disposed to face the first electrode 130a, and the other thereof may be disposed close to the second electrode 130b and thus disposed to face the second electrode 130b.

In the embodiment of FIG. 7, provided is an embodiment in which the floating electrode 125 includes a portion (e.g., the first portion 125a) facing the first electrode 130a and a portion (e.g., the second portion 125b) facing the second electrode 130b, and the portion (e.g., the first portion 125a) facing the first electrode 130a and the portion (e.g., the second portion 125b) facing the second electrode 130b are electrically connected to each other. Differently therefrom, in the embodiment of FIG. 9, provided is an embodiment in which a portion (e.g., the floating electrode 125') facing the first electrode 130a and a portion (e.g., the floating electrode 125) facing the second electrode 130b are not electrically connected to each other. Referring to FIG. 9, the first electrode 130a may be a negative (-) electrode and the second electrode 130b may be a positive (+) electrode. Differently from that illustrated in FIG. 8, it is also possible that the first electrode 130a is formed as a positive electrode and the second electrode 130b is formed as a negative electrode. In this state, it is identified that an electric field of approximately 6 to 12 volt/meter (V/m) is formed between the first electrode 130a and the second electrode 130b. Referring to FIG. 9, in case that the floating electrodes 125' facing the first electrode 130a and the second electrode 130b are not electrically connected to each other even in the case that the floating electrodes 125' facing the first electrode 130a and the second electrode 130b are arranged inside an object to be heated (e.g., the shoes S), it is identified that the same simulation result as the embodiment in which the floating electrodes are not arranged is obtained. Even though a voltage is applied to the first electrode 130a and the second electrode 130b, no charge is induced between the floating electrodes 125' facing the first electrode 130a and the second electrode 130b. Therefore, according to the embodiment illustrated in FIG. 9, even though a floating electrode is provided, the effect, in which the distance between the electrodes becomes substantially closer, may not be realized.

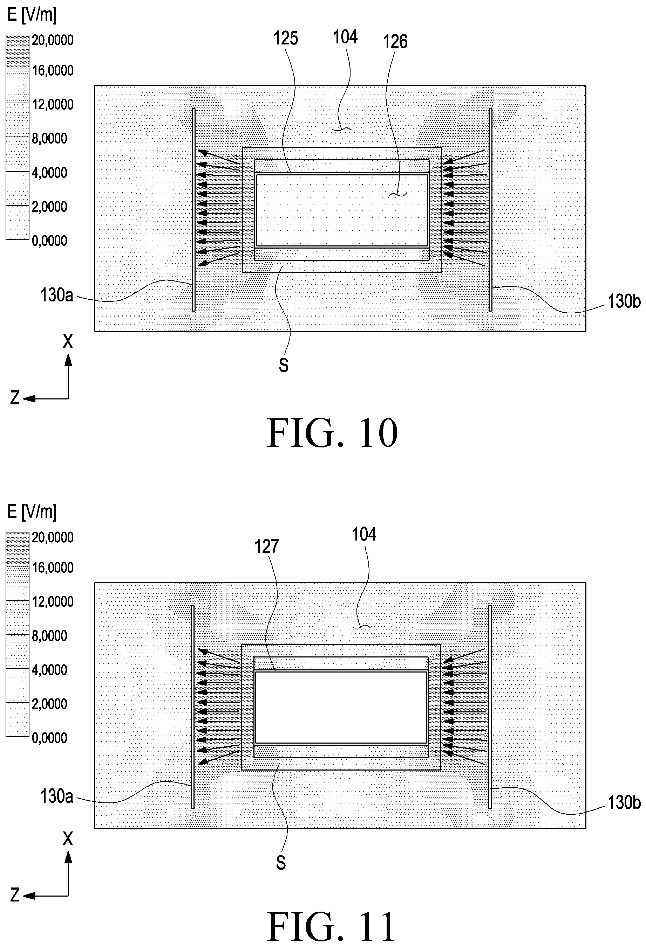

FIG. 10 is a view schematically showing an electric field formed when a voltage is applied thereto and induction heating is performed, in a state where an object to be heated and a floating structure are arranged between two electrodes according to an embodiment of the disclosure.

FIG. 10 shows a simulation result of an electric field formed in the space 104, in which the object S to be heated and the support frame 123 are arranged between the first electrode 130a and the second electrode 130b, in a state where the support frame 123 of the support member 120 is accommodated inside the object S to be heated. At this time, the first electrode 130a and the second electrode 130b may be flat plate-type electrodes, and a separate space (e.g., the spaces a1 and a2 in FIG. 7) may not be formed between an object to be heated (e.g., the shoes s) and the floating electrode 125. According to an embodiment, the floating electrode 125 may be configured as an electrode having a hollow part 126 formed therein. For example, the floating electrode 125 may be formed to have a cross section having a long rectangular shape, and may have one portion (e.g., the first portion 125a in FIG. 7) facing the first electrode 130a and the other portion (e.g., the second portion 125b in FIG. 7) facing the second electrode 130b. In addition, the other surface of the floating electrode may form an electrical movement path for charges.

Referring to FIG. 10, the first electrode 130a may be a negative (-) electrode and the second electrode 130b may be a positive (+) electrode. Differently from that illustrated in FIG. 8, it is also possible that the first electrode 130a is formed as a positive electrode and the second electrode 130b is formed as a negative electrode. In this state, it is identified that an electric field exceeding approximately 20 volt/meter (V/m) is formed around an object to be heated (e.g., the shoes S). Referring to FIG. 10, in case that the floating electrode 125 is disposed inside an object to be heated (e.g., the shoes S), it is identified that a simulation result, in which an electric field having a significantly high density is formed, is obtained, in case that a portion (e.g., the first portion 125a) facing the first electrode 130a and a portion (e.g., the second portion 125b) facing the second electrode 130b are electrically connected to each other. According to the embodiment illustrated in FIG. 10, the floating electrode 125 may be provided, and thus the effect, in which the distance between two electrodes 130a and 130b is substantially shortened, can be realized.

FIG. 11 is a view schematically showing an electric field formed when a voltage is applied thereto and induction heating is performed, in a state where an object to be heated and a floating structure are arranged between two electrodes according to an embodiment of the disclosure.

FIG. 11 shows a simulation result of an electric field formed in the space 104, in which the object to be heated (e.g., the shoes S) and the support frame 123 are arranged between the first electrode 130a and the second electrode 130b, in a state where the support frame 123 of the support member 120 is accommodated inside the object to be heated (e.g., the shoes S). At this time, the first electrode 130a and the second electrode 130b may be flat plate-type electrodes, and a separate space (e.g., the spaces a1 and a2 in FIG. 7) may not be formed between an object to be heated (e.g., the shoes s) and the floating electrode 127. According to an embodiment, the floating electrode 127 may be configured as an electrode having a hexahedral shape. In addition, the floating electrode 127 may be configured as an electrode in which the inner space thereof is filled. For example, the floating electrode 127 may be formed in a shape having a quadrilateral cross section and no hollow part, and may have one surface facing the first electrode 130a and the other surface facing the second electrode 130b.

Referring to FIG. 11, the first electrode 130a may be a negative (-) electrode and the second electrode 130b may be a positive (+) electrode. Differently from that illustrated in FIG. 8, it is also possible that the first electrode 130a is formed as a positive electrode and the second electrode 130b is formed as a negative electrode. In this state, it is identified that an electric field exceeding approximately 20 volt/meter (V/m) is formed around an object to be heated (e.g., the shoes S). Referring to FIG. 11, it is identified that a simulation result, in which an electric field having a significantly high density is formed, is obtained even in case that the floating electrode 127 having a hexahedral shape and a filled inner part is disposed inside an object to be heated (e.g., the shoes S). That is, as the embodiment illustrated in FIG. 11, the floating electrode 127 may be provided, and thus the effect, in which the distance between electrodes is substantially shortened, can be realized.

To summarize the above described description, in the electronic device 10 including two flat plate-type electrodes, in case that the floating electrode structure 125 or 127 is provided in the space between the two flat plate-type electrodes 130a and 130b, the electric field between the two flat plate-type electrodes can be strengthened. On the other hand, a floating electrode structure may be provided in the space between the two flat plate-type electrodes 130a and 130b, and thus the electrical distance between the flat plate-type electrodes 130a and 130b can also be substantially shortened. However, in this case, the floating electrode structure 125 or 127 may be configured to face each of the first electrode 130a and the second electrode 130b, but if two flat plate-type electrodes (the flat plate-type electrodes 125' in FIG. 9), which are not electrically connected to each other, are configured to face the first electrode 130a and the second electrode 130b, respectively, the expected effect cannot be obtained. Therefore. at least two surfaces (or electrodes) should be oriented toward the first electrode 130a and the second electrode 130b and the at least two surfaces (or electrodes) should be electrically connected to each other.

Referring again to FIG. 1 to FIG. 4, according to an embodiment of the disclosure, the electronic device 10 may include the processor 150, and the processor 150 may be configured to adjust intensity of voltages applied to the first electrode 130a and the second electrode 130b, based on the permittivity of an object to be heated (e.g., the shoes S). According to an embodiment, the electronic device 10 may detect the permittivity of an object to be heated (e.g., the shoes S) through at least one sensor. For example, shoes having wet soles may have permittivity higher than that of dry shoes. In this case, the processor 150 may determine that an object S to be heated needs to be heated to a higher temperature, to apply a higher voltage to the first electrode 130a and the second electrode 130b. For example, this may correspond to an operation implemented by the rapidity, luxury, or functionality button 203c in FIG. 2B pressed by a user.

At this time, the voltage applied to the electrode by the processor 150 may be in the form of an alternating current. For example, an alternating electric current having a frequency range of several MHz to several tens of MHz may be applied to the first electrode 130a and the second electrode 130b.

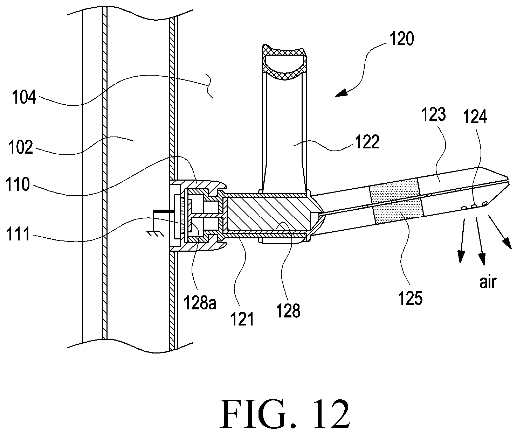

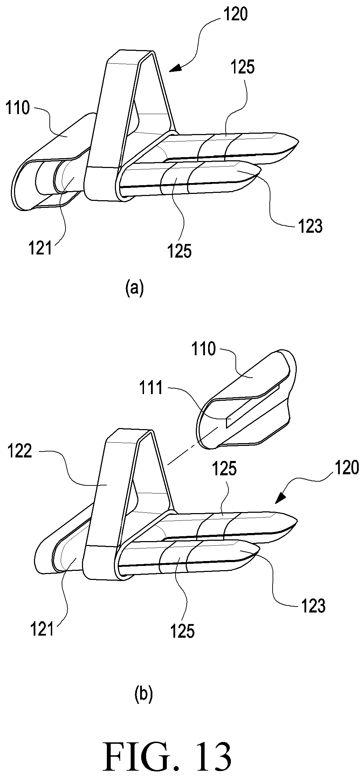

FIG. 12 is a cross-sectional view showing a state in which a support member 120 is attached to a rail structure 110 according to an embodiment of the disclosure. FIG. 13 is a view showing a state in which a support member 120 is detached from a rail structure 110 according to an embodiment of the disclosure.

According to an embodiment of the disclosure, an electrostatic prevention structure may be provided using the configuration that the support member 120 is attached to or detached from the body 100.

According to an embodiment, the support member 120 may include a conductive path 128 connected to the floating electrode 125. The support member 120 may include the conductive path 128 extending from the floating electrode 125 formed on a support frame 123 toward the base 121, and a terminal 128a of the conductive path may be formed at an end of the base 121.

Referring to FIG. 12 and FIG. 13 together, the support member 120 may be attached to and detached from the rail structure 110 in a sliding manner, may be detached from a ground terminal 111 formed in the rail structure 110 in the process of being detached from the rail structure, and may be in contact with the ground terminal 111 formed in the rail structure 110 in the process of being attached to the rail structure, thereby forming a discharging path.

A separate space is required in case that an electrostatic prevention structure is provided by wire. However, as illustrated in FIG. 12 and FIG. 13, the electronic device of the disclosure may provide an electrostatic prevention structure by using the structure in which the support member 120 is attached or detached, to effectively remove and/or prevent static electricity without separate space.

The electronic device according to an embodiment of the disclosure may be one of various types of devices. For example, the electronic device may include a shoe care device, a clothing care device, a medical appliance, as well as various home appliances. The electronic device according to embodiments of the disclosure is not limited to those described above.

It should be appreciated that the embodiments and the terms used therein are not intended to limit the technological features set forth herein to particular embodiments and the disclosure includes various changes, equivalents, and/or alternatives for a corresponding embodiment. With regard to the description of the drawings, similar reference numerals may be used to designate similar or relevant elements. A singular form of a noun corresponding to an item may include one or more of the items, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one or all possible combinations of the items enumerated together in a corresponding one of the phrases. Such terms as "a first," "a second," "the first," and "the second" may be used to simply distinguish a corresponding element from another, and does not limit the elements in other aspect (e.g., importance or order). If an element (e.g., a first element) is referred to, with or without the term "operatively" or "communicatively", as "coupled with/to" or "connected with/to" another element (e.g., a second element), it means that the element may be coupled/connected with/to the other element directly (e.g., wiredly), wirelessly, or via a third element.

As used in an embodiment of the disclosure, the term "module" may include a unit implemented in hardware, software, or firmware, and may be interchangeably used with other terms, for example, "logic," "logic block," "component," or "circuit". The "module" may be a single integrated component, or a minimum unit or part thereof, adapted to perform one or more functions. For example, according to an embodiment, the "module" may be implemented in the form of an application-specific integrated circuit (ASIC).

An embodiment of the disclosure may be implemented as software (e.g., the program 1101) including one or more instructions that are stored in a storage medium (e.g., the internal memory 1136 or external memory 1138) that is readable by a machine (e.g., the electronic device 1140). For example, a processor (e.g., the processor 1120) of the machine (e.g., the electronic device 1101) may invoke at least one of the one or more stored instructions from the storage medium, and execute it. This allows the machine to be operated to perform at least one function according to the at least one instruction invoked. The one or more instructions each may include a code generated by a complier or a code executable by an interpreter. The machine-readable storage medium may be provided in the form of a non-transitory storage medium. Herein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, the method according to an embodiment of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store TM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each element (e.g., a module or a program) of the above-described elements may include a single entity or multiple entities, and some of the multiple entities may also be separately disposed in another element. According to an embodiment, one or more of the above-described elements may be omitted, or one or more other elements may be added. Alternatively or additionally, a plurality of elements (e.g., modules or programs) may be integrated into a single element. In such a case, according to various embodiments, the integrated element may still perform one or more functions of each of the plurality of elements in the same or similar manner as they are performed by a corresponding one of the plurality of elements before the integration. According to various embodiments, operations performed by the module, the program, or another element may be carried out sequentially, in parallel, repeatedly, or heuristically, or one or more of the operations may be executed in a different order or omitted, or one or more other operations may be added.

According to an embodiment of the disclosure, provided may be an electronic device 10 including: a housing 101 configured to provide a space 104 capable of accommodating an object S to be heated; a first electrode 130a and a second electrode 130b configured to form an electric field in the space 104 in which the object S to be heated is accommodated; and a floating electrode 125 disposed between the first electrode 130a and the second electrode 130b, the floating electrode 125 configured to be positioned inside the object S to be heated based on the object S to be heated being disposed in the space 104.

According to an embodiment, the electronic device 10 may be a shoe care device configured to accommodate, in the space, shoes as an object to be heated.

According to an embodiment, a support member 120, which is configured to support the object S to be heated in the space 104 in which the object S to be heated is accommodated, may be further included therein.

According to an embodiment, based on the electronic device being a shoe care device, the support member may be a shoe tree.

According to an embodiment, the floating electrode may be formed in a form of being exposed from the support member to the outside or be formed in a form of not being exposed to the outside in a state of being disposed inside the support member.

According to an embodiment, the electronic device may include an attachable/detachable rail to allow the support member to be attached or detached.

According to an embodiment, in case that the support member is attached, the floating electrode may be electrically connected to a ground electrode provided in the attachable/detachable rail.

According to an embodiment, a case, which forms the space 104 capable of accommodating the object S to be heated, may be included therein.

According to an embodiment, at least one shelf, which is configured to partition the space 104 capable of accommodating the object S to be heated into at least two spaces, may be included therein.

According to an embodiment, the first electrode 130a and the second electrode 130b may be flat plate-type electrodes embedded in the case or the shelf.

According to an embodiment, the floating electrode 125 may be formed in a loop shape surrounding a hollow part thereof.

According to an embodiment, the floating electrode 125 may be formed in a hexahedral shape.

According to an embodiment, the floating electrode 125 may include at least two surfaces facing the first electrode and the second electrode.

According to an embodiment, a processor may be further included therein, and the electronic device may be configured to adjust intensity of voltages applied to the first electrode and the second electrode by the processor, based on the permittivity of the object to be heated.

According to an embodiment, the first electrode and the second electrode are configured such that an alternating electric current having a frequency range of several MHz to several tens of MHz may be applied to the first electrode and the second electrode.

According to an embodiment of the disclosure, provided may be an electronic device 10 including: a housing 101 including a case 102 configured to provide a space 104 capable of accommodating an object S to be heated, and at least one shelf 103 configured to partition the space 104 capable of accommodating the object S to be heated into at least two spaces; a first electrode 130a formed on a portion of the case or a portion of the shelf to form an electric field in the space 104; a second electrode 130b formed on a portion of the case or a portion of the shelf, which is opposite to the first electrode 130a; a support member 123 configured to support the object S to be heated in the space 104 in which the object S to be heated is accommodated; and a floating electrode 125 formed on the support member, the floating electrode 125 being disposed between the first electrode 130a and the second electrode 130b and configured to be positioned inside the object S to be heated based on the object S to be heated being disposed in space 104.

In the above descriptions, although specific embodiments are described in the detailed description of an embodiment of the disclosure, it will be obvious to a person skilled in the art that various changes are possible within the range without departing from the gist of the disclosure. For example, the electronic device of the disclosure is not limited to a shoe care device which cares about footwear (e.g., the shoes S) as an object to be heated, and may be applied to care about devices for various other products, including a clothing care device.

## Claims

1. An electronic device (10) comprising:
a body (100) configured to provide a space (104) capable of accommodating an object (S) to be heated;
a first electrode (130a) and a second electrode (130b) configured to form an electric field in the space (104) in which the object (S) to be heated is accommodated; and
a floating electrode (125) disposed between the first electrode (130a) and the second electrode (130b), the floating electrode (125) configured to be positioned inside the object (S) to be heated based on the object (S) to be heated being positioned in the space (104).

2. The electronic device of claim 1, wherein the electronic device (10) is a shoe care device configured to accommodate, in the space, shoes as the object to be heated.

3. The electronic device of claim 1, further comprising a support member (120) configured to support the object (S) to be heated in the space (104) in which the object (S) to be heated is accommodated.

4. The electronic device of claim 3, wherein based on the electronic device being a shoe care device, the support member is a shoe tree.

5. The electronic device of claim 3 or 4, wherein the floating electrode is formed in a form of being exposed from the support member to the outside or is formed in a form of not being exposed to the outside in a state of being disposed inside the support member.

6. The electronic device of one of claims 1 to 5, wherein the electronic device comprises a rail structure (110) configured to allow the support member to be attached or detached.

7. The electronic device of claim 6, wherein in case that the support member is attached, the floating electrode is electrically connected to a ground electrode provided in the rail structure.

8. The electronic device of one of claims 1 to 7, further comprising a case forming the space (104) capable of accommodating the object (S) to be heated.

9. The electronic device of one of claims 1 to 8, further comprising at least one shelf (103) configured to partition the space (104) capable of accommodating the object (S) to be heated into at least two spaces.

10. The electronic device of claim 9, wherein the first electrode (130a) and the second electrode (130b) are flat plate-type electrodes embedded in the case or the shelf.

11. The electronic device of one of claims 1 to 10, wherein the floating electrode (125) is formed in a loop shape surrounding a hollow part thereof.

12. The electronic device of one of claims 1 to 11, wherein the floating electrode (125) is formed in a hexahedral shape.

13. The electronic device of one of claims 1 to 11, wherein the floating electrode (125) comprises at least two portions facing the first electrode and the second electrode, and the at least two portions are electrically connected to each other.

14. The electronic device of claims 1 to 13, further comprising a processor,
wherein the electronic device is configured to adjust intensity of voltages applied to the first electrode and the second electrode by the processor, based on the permittivity of the object to be heated.

15. **16.** The electronic device of claim 1, wherein the first electrode and the second electrode are configured such that an alternating electric current having a frequency range of several MHz to several tens of MHz is applied to the first electrode and the second electrode.
